(19) Europäisches Patentamt
European Patent Office
Office européen des brevets

(11) EP 1 726 304 A1

(12) **EUROPEAN PATENT APPLICATION**
published in accordance with Art. 158(3) EPC

(43) Date of publication:
**29.11.2006 Bulletin 2006/48**

(21) Application number: **05720613.8**

(22) Date of filing: **11.03.2005**

(51) Int Cl.:
***A61K 31/435*** *(2006.01)* *C07D 453/02* *(2006.01)*

(86) International application number:
**PCT/JP2005/004342**

(87) International publication number:
**WO 2005/087231 (22.09.2005 Gazette 2005/38)**

(84) Designated Contracting States:
**AT BE BG CH CY CZ DE DK EE ES FI FR GB GR HU IE IS IT LI LT LU MC NL PL PT RO SE SI SK TR**

(30) Priority: **16.03.2004 US 553123 P**
**28.04.2004 JP 2004133283**

(71) Applicant: **Astellas Pharma Inc.**
**Tokyo 103-8411 (JP)**

(72) Inventors:
- **INAKOSHI, Masatoshi,**
**Astellas Pharma Inc.**
**Tokyo 103-8411 (JP)**
- **ISHII, Yusuke,**
**Astellas Pharma Inc.**
**Tokyo 103-8411 (JP)**

(74) Representative: **HOFFMANN EITLE**
**Patent- und Rechtsanwälte**
**Arabellastrasse 4**
**81925 München (DE)**

(54) **SOLIFENACIN-CONTAINING COMPOSITION**

(57) To provide a novel method for producing a composition comprising solifenacin or a salt thereof, and a composition comprising solifenacin or a salt thereof as produced by the method, wherein an optionally substituted lower alkyl is added to the 2-position of the quinuclidine of solifenacin. The composition of the present invention contains a highly pure solifenacin, while the unexpected compounds specific to the method in an extremely low content, so that it has very preferable properties as a bulk for pharmaceutical products.

EP 1 726 304 A1

## Description

### Technical Field

The present invention relates to a novel method for producing solifenacin or a salt thereof, which is useful as a medicament, particularly as a muscarine $M_3$ receptor antagonist, more specifically as a therapeutic agent for urological diseases such as pollakiuria and urinary incontinence due to hyperactive bladder, as well as a composition comprising solifenacin or a salt thereof as obtained by the method, which contains a specific solifenacin derivative.

### Background Art

Solifenacin or a salt thereof is a compound known as a muscarine $M_3$ receptor antagonist (see patent reference 1, non-patent reference 1, non-patent reference 2, non-patent reference 3). Clinical tests thereof as a therapeutic agent of pollakiuria and incontinence of urine due to hyperactive bladder have been under way. Additionally, it is reported that the compound has effects on interstitial cystitis (patent reference 2), tension relaxation of ciliary muscle (patent reference 3), hypersensitive colon syndrome (non-patent reference 4), and so on.

Further, the chemical name of solifenacin is (1S,3'R)-quinuclidin-3'-yl 1-phenyl-1,2,3,4-tetrahydroisoquinoline-2-carboxylate and has the following chemical structure.

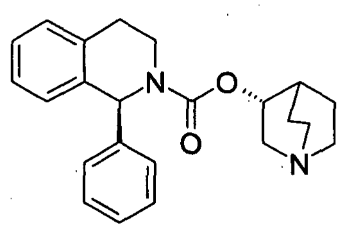

As a specific method for producing solifenacin or a salt thereof, the patent reference 1 specifically discloses the method depicted by the following scheme. Other than this report, there is no reference disclosing any detailed method for producing solifenacin or a salt thereof.

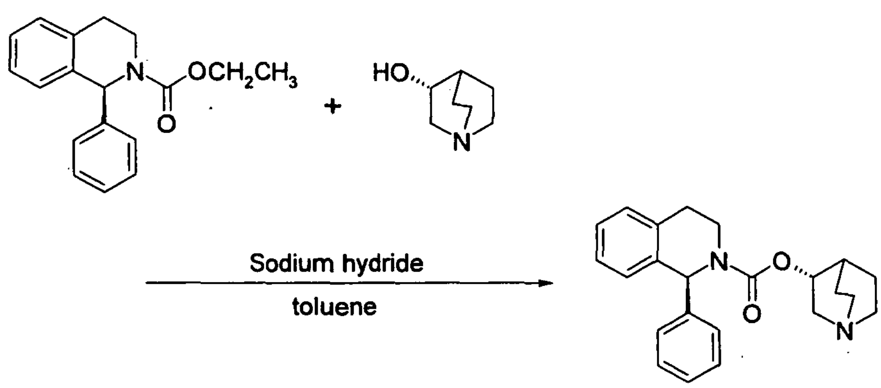


Additionally, the production method shown by the following scheme is known as a method for producing a compound with a similar structure. However, no example exists where the production method is applied to solifenacin production (patent reference 4).

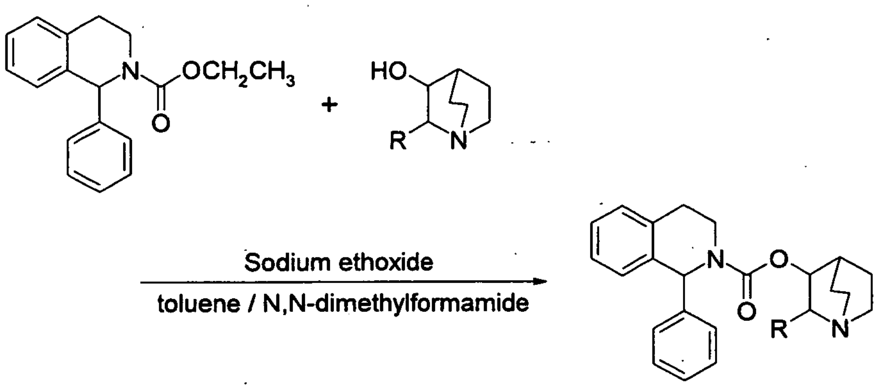


[in the formula, R represents methyl or ethyl].

Further, there is a report about the studies on the crystallization conditions for solifenacin succinate by controlling the particle size of solifenacin succinate to improve the fluidity thereof with a significant influence on the handling in the formulation thereof and on the equipment for the bulk. However, the report simply tells about the crystallization conditions for improving the fluidity. The report does not suggest or mention the composition of a pharmaceutical composition comprising solifenacin succinate (non-patent reference 5).

Additionally, it is required that the bulk as the active ingredient used as the raw material of medicaments should have high purity. In the Case Tokyo High Court Heisei 9 (Gyo ke) No. 302 determined on February 27, 2000, the decision remarks that "when diagnostic agents and therapeutic agents contain even a trace amount of impurities above the acceptable limits, a possibly of an adverse influence on diagnosis and therapeutic treatment cannot be denied, which is obviously belong to the technical field which the present invention belongs." That is, for the medicament, it is a technical common sense that it is important to obtain a bulk having a high purity with impurities as low as possible.

[Patent reference 1] Specification of EP Patent No. 801067
[Patent reference 2] Pamphlet of International Publication WO 2003/6019
[Patent reference 3] Published Japanese patent application JP-A-2002-104968
[Patent reference 4] Published Japanese patent application JP-A-2003-267977
[Non-patent reference 1] Current Opinion in Central & Peripheral Nervous System International Drugs, 2000, Vol. 2, No.3, p. 321-325
[Non-patent reference 2] Drugs of the Future, 1999, Vol.24, No.8, p.871-874
[Non-patent reference 3] Naunyn-Schmiedeberg's Archives of Pharmacology, 2002, Vol.366, No.2, p.97-103
[Non-patent reference 4] Japanese Journal of Pharmacology, 2001, Vol. 86, No.3, p.281-288
[Non-patent reference 5] The Abstract of Lectures at the Memorial Symposium for the Foundation and Establishment of Japan Process Chemistry Association (held on July 4 to July 5, 2002), p.85-86.

Disclosure of the Invention

Problems to Be Solved by the Invention

Under such circumstances, it is desired to provide a method for easily producing solifenacin or a salt thereof which is useful as a medicament, as well as a composition containing a highly pure solifenacin or a salt thereof.

The inventors made extensive studies on a method for producing solifenacin. As a result, the inventors found that solifenacin or a salt thereof can be obtained by production methods including a new production method shown by the following scheme and also found unexpectedly that a compound specific to the production method was essentially generated at less than 1% to solifenacin, wherein an optionally substituted lower alkyl is added to the 2'-position of solifenacin in producing solifenacin or a salt thereof by the production method, thereby obtaining a solifenacin-containing composition comprising such a compound.

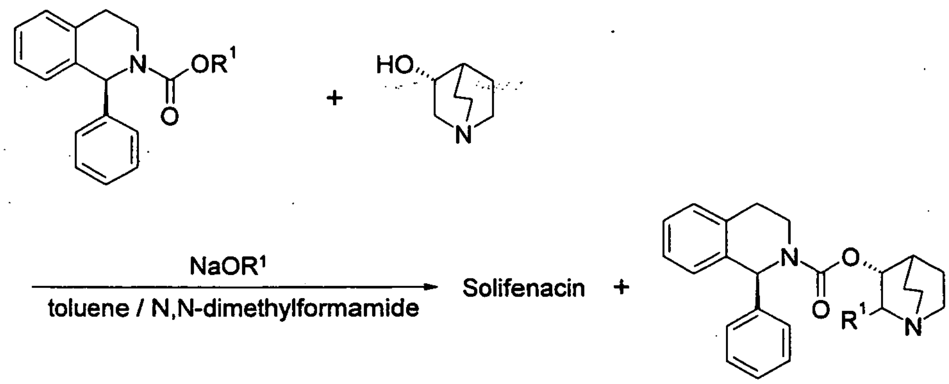


[in the formula, $R^1$ represents an optionally substituted lower alkyl].

Compared to the production method described in the patent reference 1, this production method is characterized in that, instead of the use of sodium hydride having disadvantages such as a combustion risk and contaminations of mineral oil contained therein, an alkali metal lower alkoxide without such disadvantages is used, so that it is suited for the industrial production.

In accordance with the present invention, there is provided a composition comprising solifenacin or a salt thereof, particularly a pharmaceutical composition comprising solifenacin or a salt thereof, where a compound represented by the formula (I) including optical isomers thereof

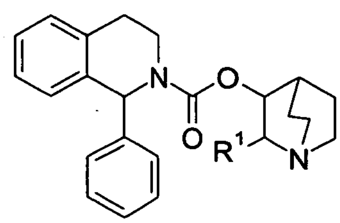 (I)

[in the formula, $R^1$ represents an optionally substituted lower alkyl; the same applies hereinafter] is contained at a total of less than 1%, preferably a total of less than 0.7%, more preferably a total of less than 0.1% to solifenacin or a salt thereof. In this specification, the content ratios of a compound represented by the compound (I) or a salt thereof all express the content ratios of a total of various types of optical isomers thereof. Further, $R^1$ is preferably ethyl and, as another embodiment, is preferably methyl or benzyl.

In accordance with the present invention, a composition comprising solifenacin or a salt thereof is provided, which is produced by a production method comprising the reaction between a compound represented by the formula (II)

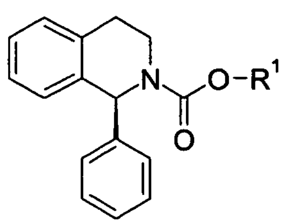 (II)

[in the formula, $R^1$ represents a lower alkyl] and (R)-quinuclidin-3-ol in the presence of an alkali metal lower alkoxide.

Furthermore, in accordance with the present invention, a method for producing the composition comprising solifenacin is provided, comprising the reaction between a compound represented by the formula (II) and (R)-quinuclidin-3-ol in the presence of an alkali metal lower alkoxide.

Effects of the Invention

The a novel method for producing a composition comprising solifenacin or a salt thereof according to the present invention is industrially suited because, instead of sodium hydride having disadvantages such as combustion risk and contaminations of mineral oil contained therein, the alkali metal lower alkoxide without any such disadvantages is used. Additionally, the composition comprising solifenacin or a salt thereof as produced by such a method contains the bulk of solifenacin or a pharmaceutically acceptable salt thereof at high purity and contains unexpected compounds specific to the method at a content as low as less than 1%. Thus, the composition has very preferable properties as a bulk for pharmaceutical products.

Brief Description of Drawings

[Fig. 1] A chart of the composition of the solifenacin-containing ethyl acetate solution obtained in Example 1, as measured by HPLC. A peak at a retention time of about 11.9 minutes shows solifenacin; peaks at retention times of about 15.5 minutes, about 15.9 minutes and about 17.6 minutes show individual optical isomers of the compound X. Furthermore, the peak at a retention time of about 25.8 minutes shows toluene contained in the solifenacin-containing ethyl acetate solution obtained in Example 1.

[Fig. 2] A chart of the composition of the solifenacin succinate-containing ethyl acetate solution obtained in Example 2, as measured by HPLC. A peak at a retention time of about 11.8 minutes shows solifenacin; peaks at retention times of about 15.2 minutes and about 17.2 minutes show individual optical isomers of compound X.

[Fig. 3] A chart of the composition of the solifenacin succinate-containing ethyl acetate solution obtained in Example 3, as measured by HPLC. A peak at a retention time of about 15.7 minutes shows solifenacin; a peak at a retention time of about 21.5 minutes shows compound D.

Best Mode for Carrying Out the Invention

In this specification, the term "lower alkyl" means a linear or branched $C_{1-6}$ alkyl and specifically includes for example methyl, ethyl, propyl, butyl, pentyl or hexyl. Preferably, the lower alkyl is methyl, ethyl and propyl. Particularly preferable is ethyl.

Additionally, substituents acceptable in the "optionally substituted lower alkyl" as $R^1$ include any group generally acceptable as the substituent for the lower alkyl. Illustrative examples thereof include phenyl.

The term "alkali metal lower alkoxide" means a salt between an alcohol corresponding to the above-described lower alkyl with an alkali metal, where the alkali metal includes, for example, lithium, sodium and potassium, preferably sodium and potassium and more preferably sodium. The "alkali metal alkoxide" specifically includes, for example, sodium methoxide, sodium ethoxide, sodium propoxide, sodium butoxide, sodium benzyloxide, potassium methoxide and potassium ethoxide.

The term "a salt thereof" in the "solifenacin or a salt thereof" means any salt of solifenacin with a pharmaceutically acceptable acid and specifically includes acid addition salts of solifenacin with inorganic acids such as hydrochloric acid and sulfuric acid; or organic acids such as acetic acid, oxalic acid and malonic acid.

All the content ratios described in the present specification represent the area ratios by HPLC analysis when the area of solifenacin succinate is defined as 100%. The content ratios are measured under the following conditions for the HPLC analysis or under the similar conditions.

Furthermore, the composition of the present invention also encompasses compositions comprising so-called labeled compounds produced by substituting a part or the whole of atoms constituting solifenacin or a compound represented by the formula (I) with radioisotopes.

The composition of the present invention can be produced by the production method comprising reacting a compound represented by the above formula (II) with (R)-quinuclidin-3-ol in the presence of an alkali metal lower alkoxide or a variation method thereof. The reaction can preferably be carried out in a solvent inactive to the reaction, such as aromatic hydrocarbons (e.g., benzene, toluene, xylene and methitylene); ethers (e.g., diethyl ether, tetrahydrofuran, and dioxane); halogenated hydrocarbons (e.g., dichloromethane, 1,2-dichloroethane and chloroform); N,N-dimethylformamide; N,N-dimethylacetoamide; dimethylsulfoxide, or a mixture solvent thereof, using the compound represented by the above formula (II) and (R)-quinuclidin-3-ol at an equal mole to an excess amount of either one of them, under cooling to at ambient temperature, at ambient temperature to under heating, or at ambient temperature to under reflux, while the solvent is distilled off under reflux. The alkali metal lower alkoxide can be used at a catalytic amount to an excess amount. The alkali metal lower alkoxide is preferably used at 0.1 to 1.2 equivalent amounts, preferably 0.15 to 0.4 equivalent amount.

Example

The present invention is now described specifically in the following Examples. However, the present invention is not limited to the Examples at all.

Reference Example 1

Production of seed crystal of solifenacin succinate

60 liters of water and then 23.8 kg of potassium carbonate were added to a mixture of 30.0 kg of (S)-1-phenyl-1,2,13,4-tetrahydroisoquinoline and 300 liters of toluene. The resulting mixture was cooled to 10˚C, to which 18.7 kg of ethyl chloroformate was subsequently dropwise added, for agitation for one hour. After the aqueous layer was separated, the resulting organic layer was rinsed with 150 liters of water. The organic layer was further rinsed with 150 liters of water, from which the solvents were distilled off under reduced pressure.

360 liters of toluene and 40 liters of N,N-dimethylformamide were added to the resulting residue, to which 21.6 kg of (R)-quinuclidin-3-ol and 2.89 kg of sodium ethoxide were added at ambient temperature. While distilling off the solvents, the mixture was heated for 8 hours. 200 liters of water was added to the reaction mixture, and cooled to ambient temperature, from which the aqueous layer was separated. The organic layer was rinsed with 200 liters of water. The organic layer was further extracted with 47.6 kg of conc. hydrochloric acid and 360 liters of water. Then, a mixture of 72.5 kg of potassium carbonate and 400 liters of water was added to the resulting aqueous layer, for extraction into 400 liters of ethyl acetate to give an organic phase containing (1S,3'R)-quinuclidin-3'-yl 1-phenyl-1,2,3,4-tetrahydroisoquinoline-2-carboxylate (referred to as "solifenacin" hereinafter).

After the resulting organic layer was rinsed with 100 liters of water, the solvents therein were distilled off. To the resulting residue were added 450 liters of ethyl acetate, 90 liters of ethanol and 14.6 kg of succinic acid, for dissolution under heating. The solution was then cooled to 0˚C, to filter and recover the deposited crystal, which was then rinsed with 80 liters of ethyl acetate and dried under reduced pressure, to obtain solifenacin succinate at 46.40 kg.

Example 1

Production of solifenacin

A mixture of 360 liters of water and 83.2 kg of potassium carbonate was added to a mixture of 120 kg of (S)-1-phenyl-1,2,3,4-tetrahydroisoquinoline and 600 liters of toluene. The resulting mixture was cooled to 10˚C, to which 65.3 kg of ethyl chloroformate was subsequently dropwise added, for agitation at 25˚C for 2 hours. After the aqueous layer was separated, the organic layer was rinsed with 360 liters of water. After 290 liters of the solvents were distilled off under reduced pressure, 1320 liters of toluene and 81 liters of N,N-dimethylformamide were further added, to which 87.5 kg of (R)-quinuclidin-3-ol and 7.8 kg of sodium ethoxide were added at ambient temperature. While distilling off the solvents, the mixture was heated for 8 hours. 480 liters of toluene and 400 liters of water were added to the reaction solution, which was then cooled to ambient temperature, from which the aqueous layer was separated. The resulting organic layer was rinsed with 400 liters of water. The organic layer was further extracted with 77.4 kg of conc. hydrochloric acid and 440 liters of water. Then, a mixture of 126.8 kg of potassium carbonate and 320 liters of water was added to the resulting aqueous layer, for extraction into 810 liters of ethyl acetate. After the organic layer was rinsed with 160 liters of water, 160 liters of ethanol and 240 liters of ethyl acetate were added to the organic layer. 820 liters of the solvents were distilled off from the solution at atmospheric pressure, to obtain 527.8 kg of an ethyl acetate solution of a solifenacin-containing composition containing 2-ethylquinuclidine-3-yl 1-phenyl-1,2,3,4-tetrahydroisoquinoline-2-carboxylate (referred to as "compound X" hereinafter).

Example 2

Production of solifenacin succinate-containing composition

To 261.0 kg of the ethyl acetate solution of a solifenacin-containing composition as obtained in Example 1 were added 140 liters of ethanol, 120 liters of ethyl acetate and 31.1 kg of succinic acid, followed by dissolution under heating. 12 liters of ethanol and 28 liters of ethyl acetate were added, which was then cooled to 50˚C. 9.11 g of solifenacin succinate produced in the same manner as in Reference Example 1 was added. The resulting mixture was cooled to 0˚C, and the precipiated crystals were collected by filtration. The resulting crystals were rinsed with 190 liters of ethyl acetate, and dried under reduced pressure, to obtain 87.82 kg of a solifenacin succinate-containing composition containing compound X.

Example 3

Production of solifenacin-containing composition

In a mixture of 90 mL of toluene and 4.5 mL of DMF and in the presence of 0.36 g of sodium methoxide, 9.00 g of methyl (S)-1-phenyl-1,2,3,4-tetrahydroisoquinoline-2-carboxylate as prepared in a method according to the first paragraph of Example 1 and 5.14 g of (R)-quinuclidin-3-ol were reacted to each other for 8 hours, while the solvent was distilled off, to obtain a solifenacin solution containing composition containing 2-methylquinuclidin-3-yl 1-phenyl-1,2,3,4-tetrahydrolsoquinoline-2-carboxylate (referred to as "compound Y" hereinafter).

Example 4

Production of solifenacin-containing composition

To a mixture of 125 mL of toluene, 19.8 g of potassium carbonate and 75 ml of water, were added 25.0 g of (S)-1-phenyl-1,2,3,4-tetrahydroisoquinoline and 24.5 g of benzylchloride carbonate, followed by stirring at 20˚C for 4 hours. The organic layer was rinsing with 75 mL of water. The resulting organic layer was concentrated under reduced pressure and subjected to purification with silica gel column chromatography and drying to obtain 38.0 g of benzyl (S)-1-phenyl-1,2,3,4-tetrahydroisoquinoline-2-carboxylate.
($^1$H-NMR (DMSO-$d_6$, tetramethylsilane internal standard): δ 2.73-2.83 (1H,m), 2.84-2.94 (1H,m), 3.31-3.41 (1H, m), 3.86-3.96 (1H, m), 5.12 (1H, d, J=12.8Hz), 5.18 (1H, d, J=12.8Hz), 6.28 (1H,s), 7.10-7.38 (14H, m).
Mass spectrum: m/z = 344$[M+H]^+$(FAB))

In a mixture of a sodium benzyl alkoxide obtained from 0.19 g of benzyl alcohol and 0.04 g of metallic sodium, 15 mL of toluene, and 0.75 mL of DMF, 1.33 g of (R)-quinuclidin-3-ol and 3.00 g of benzyl (S)-1-phenyl-1,2,3,4-tetrahydroisoquinoline-2-carboxylate were reacted to each other for 8 hours, while distilling off the solvent, to obtain 1.38 g of solifenacin containing 2-benzylquinuclidin-3-yl 1-phenyl-1,2,3,4-terahydroisoquinoline-2-carboxylate (to be referred to as "compound Z" hereinafter).

The compositions of the compositions obtained in the following Examples were measured as follows. In this regard, because a phosphate buffer was used as the mobile phase, each compound was detected as a basic substance in which addition salt had been removed.

(Method for assaying compound X)

0.05 g of a composition obtained was dissolved in solution P (solution P was was preliminarily prepared by dissolving 8.7 g of dipotassium hydrogen phosphate in water to give a 1000 mL solution, adding phosphoric acid to adjust the resulting solution to pH 6.0, and then adding 300 mL of acetonitrile to a 700 mL portion of the resulting pH-adjusted solution) to a final total volume of 100 mL (defined as sample solution). To 1 mL of the sample solution was further added the solution P, to a final total volume of 100 mL (defined as standard solution). 10 μl each of the sample solution and the standard solution was tested by liquid chromatography under the following conditions, to measure the area of each peak of the individual solutions by the automatic integral method. By the following equation, the amount of impurities was calculated.

The measured data of Example 1 is shown in Fig. 1 and the measured data of Example 2 is shown in Fig. 2.

$$\text{Content ratio (\%) of individual impurities} = ADTi/ADS$$

[in the formula, ADTi represents the peak area of individual impurities in the sample solution, while ADS represents the peak area of solifenacin in the standard solution.]

| <Test conditions> | |
|---|---|
| Detector: | Ultraviolet absorption photometer (wavelength for measurement: 210 nm) |
| Column: | Develosil ODS-UG-5 (150 × 4.6 mm ID, manufactured by Nomura Chemical) or an equivalent one |
| Column temperature: | 40˚C |

(continued)

<Test conditions>

| | |
|---|---|
| Mobile phase: | adding phosphoric acid to a solution of 8.7 g of dipotassium hydrogen phosphate dissolved in water to 1000 mL to adjust the resulting solution to pH 6.0 and then adding 200 mL of acetonitrile, 100 mL of 2-propanol and 50 mL of methanol to 650 mL portion of the resulting pH-adjusted solution. |
| Flow rate: | about 1 mL/minute |

(Method for assaying compound Y)

0.01 g of a composition obtained was dissolved in solution P to a final total volume of 10 mL (defined as sample solution). 10 μl of the sample solution was tested by liquid chromatography under the following conditions, to measure the area of the peak by the automatic integral method.

The measured data of Example 3 is shown in Fig. 3.

<Test conditions>

| | |
|---|---|
| Detector: | Ultraviolet absorption photometer (wavelength for measurement: 210 nm) |
| Column: | Develosil ODS-UG-5 (150 × 4.6 mm ID, manufactured by Nomura Chemical) |
| Column temperature: | 40°C |
| Mobile phase: | solution P |
| Flow rate: | about 1 mL/minute |

(Method for assaying compound Z)

0.03 g of a composition obtained was dissolved in solution P to a final total volume of 10 mL (defined as sample solution). To 1 mL of the sample solution was further added solution P to a final volume of 200 mL (defined as a standard solution). 20 μl each of the sample solution and the standard solution were tested by liquid chromatography under the following conditions to measure the area of each peak of the individual solutions by the automatic integral method. By the following equation, the amount of impurities was calculated.

**Content ratio (%) of individual impurities = ATi/AS/2**

[in the formula, ATi represents the peak area of individual impurities in the sample solution, while AS represents the peak area of solifenacin in the standard solution.]

<Test conditions>

| | |
|---|---|
| Detector: | Ultraviolet absorption photometer (wavelength for measurement: 210 nm) |
| Column: | ODS-A, A-302 (150 × 4.6 mm ID, manufactured by YMC) |
| Column temperature: | 40°C |
| Mobile phase: | solution P |
| Flow rate: | about 1 mL/minute |

In Table 1, the content ratios (in a total amount of optical isomers) of compound X, compound Y or compound Z in the in the composition containing solifenacin or a salt thereof as obtained in the Examples described above are shown when solifenacin or a salt thereof is defined as 100%.

(Table 1)

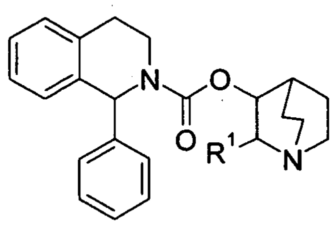

| | | $R^1$ | Content ratio |
|---|---|---|---|
| Example 1 | Compound X | ethyl | 0.67% |
| Example 2 | Compound X | ethyl | 0.08% |
| Example 3 | Compound Y | ethyl | 0.20% |
| Example 4 | Compound Z | benzyl | 0.07% |

Industrial applicability

The aforementioned results indicate that a novel method for producing a composition comprising solifenacin or a salt thereof is established and that the method is a novel method industrially suitable, where instead of sodium hydride with disadvantages such as combustion risk and contaminations of mineral oil contained therein, the alkali metal lower alkoxide without any such disadvantages is used. Additionally, the composition comprising solifenacin or a salt thereof as produced by the method of the invention contains the drug substance of solifenacin or a pharmaceutically acceptable salt thereof at high purity and contains unexpected compounds specific to the method at a content as small as less than 1%. Thus, the composition has very preferable properties as a drug substance for pharmaceutical products.

## Claims

1. A composition comprising solifenacin or a salt thereof, wherein a compound represented by the formula (I) including optical isomers thereof

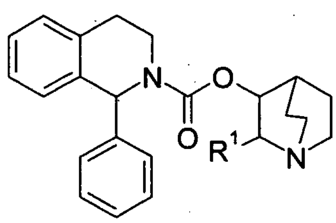

(I)

[in the formula, $R^1$ represents an optionally substituted lower alkyl; the same applies hereinbelow] or a salt thereof is contained in a total amount of less than 1% to solifenacin or a salt thereof.

2. A composition according to claim 1, wherein a compound represented by the formula (I) including optical isomers thereof or a salt thereof is contained in a total amount of less than 0.7% to solifenacin or a salt thereof.

3. A composition according to claim 1, where a compound represented by the formula (I) including optical isomers thereof or a salt thereof is contained in a total amount of less than 0.1% to solifenacin or a salt thereof.

4. A composition according to any one of claims 1, 2 and 3, wherein $R^1$ is ethyl.

5. A composition according to any one of claims 1, 2 and 3, wherein $R^1$ is methyl or benzyl

**6.** A composition according to any one of claims 1 to 5, as produced by a production method comprising reacting a compound represented by the formula (II)

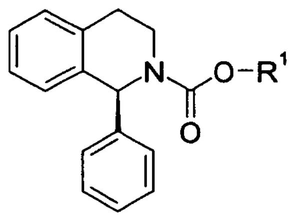

(II)

[in the formula, $R^1$ represents a lower alkyl] and (R)-quinuclidin-3-ol in the presence of an alkali metal lower alkoxide.

**7.** A method for producing a composition according to any one of claims 1 to 5, comprising reacting a compound represented by the formula (II) according to claim 6 and (R)-quinuclidin-3-ol in the presence of an alkali metal lower alkoxide.

**8.** A composition according to any one of claims 1 to 5, which is a pharmaceutical composition.

**9.** A composition according to claim 6, which is a pharmaceutical composition.

[Fig. 1]

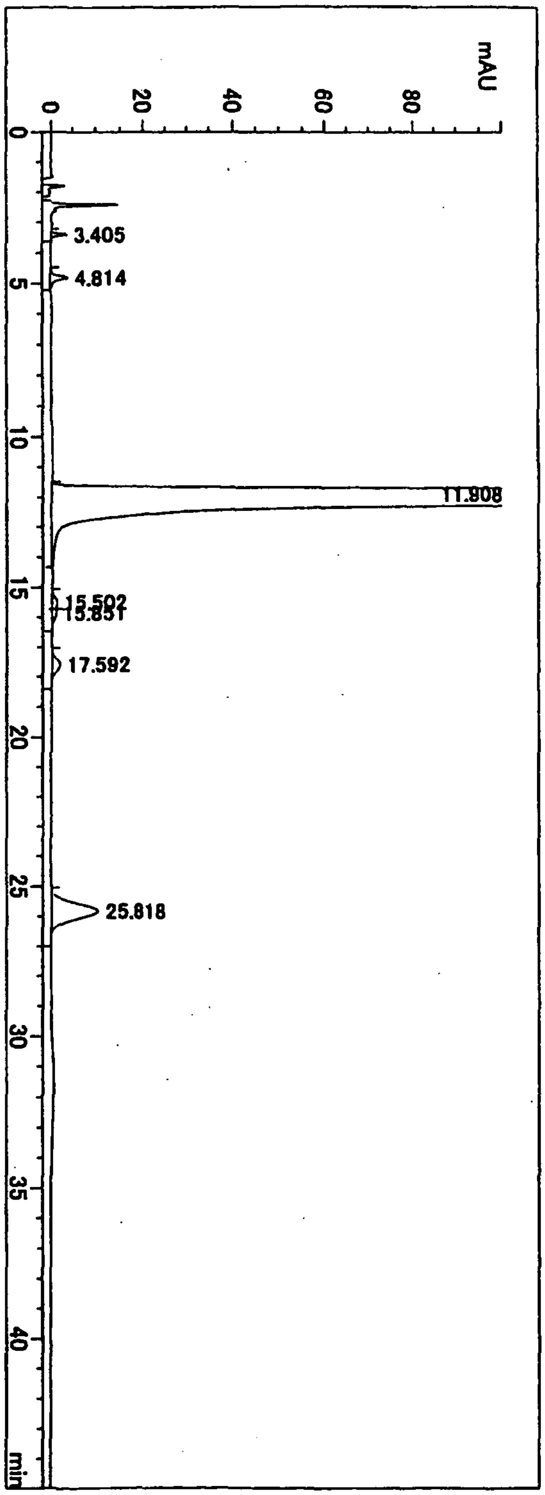
mAU
0
20
40
60
80
0
5
10
15
20
25
30
35
40
min
3.405
4.814
11.908
15.502
15.851
17.592
25.818

[Fig. 2]

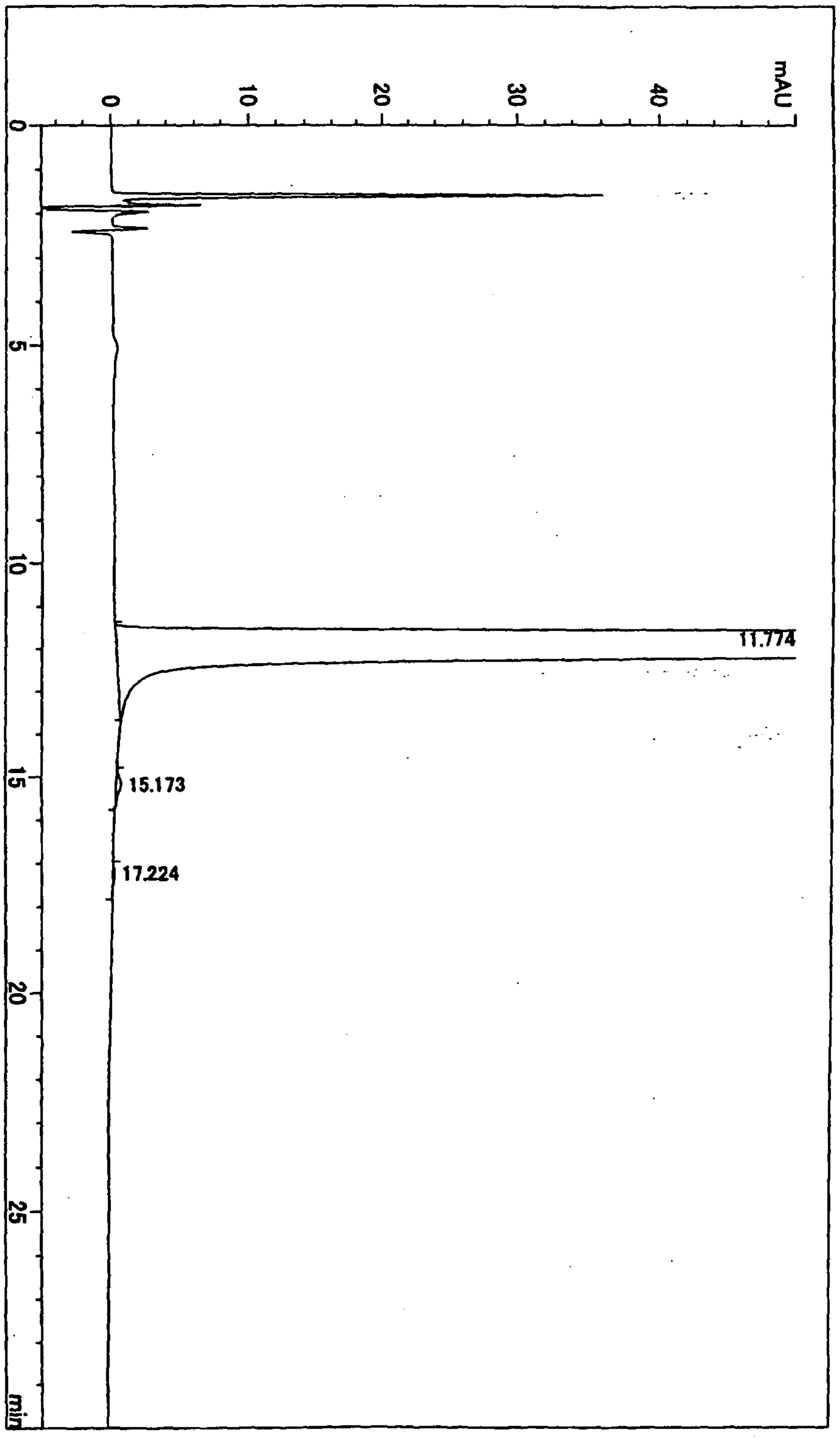

[Fig. 3]

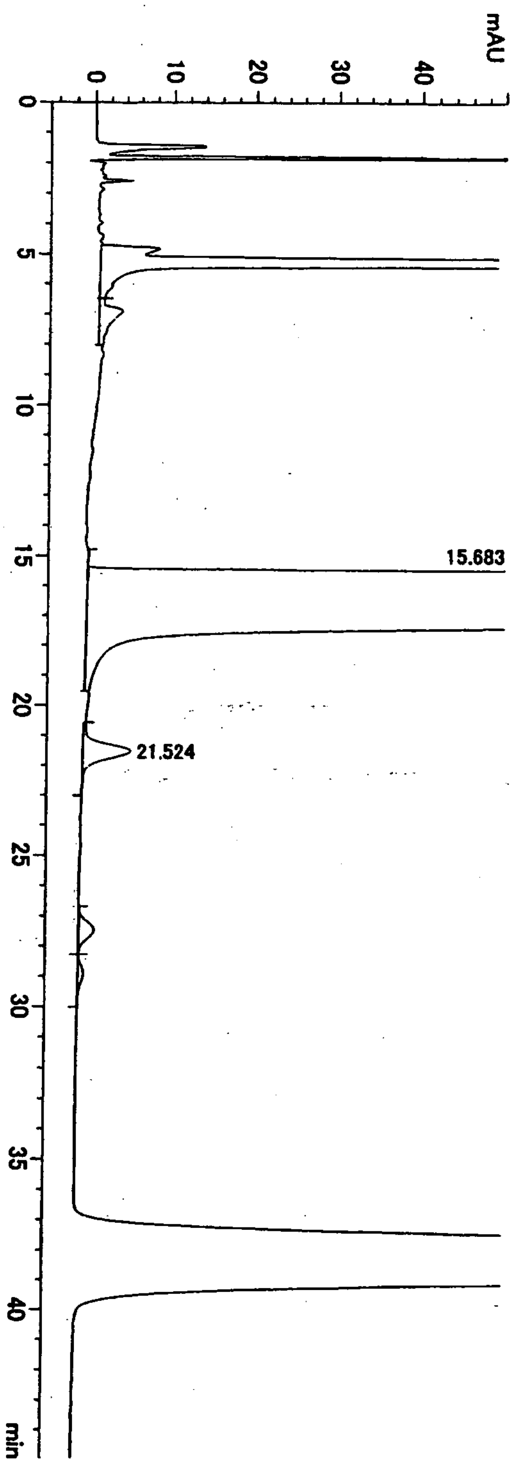

**INTERNATIONAL SEARCH REPORT**

International application No.
PCT/JP2005/004342

A. CLASSIFICATION OF SUBJECT MATTER
Int.Cl[7] A61K31/435//C07D453/02

According to International Patent Classification (IPC) or to both national classification and IPC

B. FIELDS SEARCHED

Minimum documentation searched (classification system followed by classification symbols)
Int.Cl[7] A61K31/435//C07D453/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
REGISTRY/CA(STN)

C. DOCUMENTS CONSIDERED TO BE RELEVANT

| Category* | Citation of document, with indication, where appropriate, of the relevant passages | Relevant to claim No. |
|---|---|---|
| X | WO 96/20194 A1 (Yamanouchi Pharmaceutical Co., Ltd.), 04 July, 1996 (04.07.96), Example 10; page 12 & EP 801067 A1 & US 6017927 A1 | 1-9 |
| A | JP 2003-267977 A (Yamanouchi Pharmaceutical Co., Ltd.), 25 September, 2003 (25.09.03), Full text (Family: none) | 1-9 |

☐ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 April, 2005 (13.04.05) | 26 April, 2005 (26.04.05) |
| Name and mailing address of the ISA/ Japanese Patent Office | Authorized officer |
| Facsimile No. | Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 801067 A
- WO 20036019 A
- JP 2002104968 A
- JP 2003267977 A


### Non-patent literature cited in the description


- *Case Tokyo High Court Heisei,* 27 February 2000, vol. 9 (302
- *Current Opinion in Central & Peripheral Nervous System International Drugs,* 2000, vol. 2 (3), 321-325
- *Drugs of the Future,* 1999, vol. 24 (8), 871-874
- *Naunyn-Schmiedeberg's Archives of Pharmacology,* 2002, vol. 366 (2), 97-103
- *Japanese Journal of Pharmacology,* 2001, vol. 86 (3), 281-288
- *The Abstract of Lectures at the Memorial Symposium for the Foundation and Establishment of Japan Process Chemistry Association,* 04 July 2002, 85-86